# EUROPÄISCHE PATENTANMELDUNG

(11) **EP 0 664 336 A1**
(43) Veröffentlichungstag der Anmeldung: **26.07.1995**
(21) Anmeldenummer: 95100331.8
(22) Anmeldetag: 12.01.1995
(51) Int. Cl.: C12N 5/12, C12P 21/08, C12Q 1/04, G01N 33/577

(54) **Saccharomyces-spezifische Antigene und Antikörper, deren Herstellung und Verwendung**

(30) Priorität: 25.01.1994 DE 4402065
(71) Anmelder: BEHRINGWERKE Aktiengesellschaft, 35001 Marburg (DE)
(72) Erfinder: Bröker, Michael, Dr., D-35041 Marburg (DE); Harthus, Hans-Peter, Dr., D-35041 Marburg (DE)

(57) **Zusammenfassung**

Die Erfindung betrifft Saccharomyces cerevisiae-, Saccharomyces bayanus-, Saccharomyces paradoxus- und Saccharomyces pastorianus-spezifische Antigene und dagegen gerichtete Antikörper. Diese Antigene und Antikörper können zur Identifizierung und Quantifizierung der genannten Saccharomycesarten eingesetzt werden.

## Beschreibung

Die Erfindung betrifft Saccharomyces cerevisiae-, Saccharomyces bayanus-, Saccharomyces paradoxus- und Saccharomyces pastorianus-spezifische Antigene und dagegen gerichtete Antikörper. Diese Antigene und Antikörper können zur Identifizierung und Quantifizierung der genannten Saccharomycesarten eingesetzt werden.

Nach van der Walt (J. P. van der Walt [1970], Genus 16, pp. 555-718) sind der Gattung Saccharomyces 41 Arten zugeordnet. In der Zwischenzeit wurde jedoch eine Reihe dieser Arten anderen Gattungen zugeordnet bzw. sind Umbenennungen innerhalb der Gattung Saccharomyces erfolgt. Gegenwärtig sind innerhalb der Gattung Saccharomyces 10 Arten akzeptiert (Barnett, J. A. [1992] Yeast, 8, pp. 1-23).

Diese 10 Saccharomycesarten sind: S. bayanus, S. castellii, S. cerevisiae, S. dairensis, S. exiguus, S. kluyveri, S. paradoxus, S. pastorianus. S. servazzii, S. unisporus.

In Zukunft wird auch diese Klassifizierung wieder Änderungen erfahren, da die Regeln und Kriterien, nach denen die taxonomische Einstufung von Hefearten vorgenommen wird, Änderungen unterworfen sind. Z. B. könnte man argumentieren, daß die Arten S. bayanus, S. paradoxus und S. pastorianus gar nicht als eigenständige Arten zu sehen sind, sondern vielmehr als Varietäten von S. cerevisiae.

Es bleibt letzlich festzustellen, daß die Systematik der Hefegattung Saccharomyces von praktischer Bedeutung ist, da einige Arten industriell, insbesondere in der Bier- und Weinindustrie, in der Backindustrie und in der Biotechnologie genutzt werden, und deshalb über das akademische Interesse hinaus klare Zuordnungen erfolgen müssen, die allgemein akzeptiert werden.

Folgende Terminologie wird in Anlehnung an Naumov et al. (1992) Yeast, 8, pp. 599-612 zur Beschreibung vorliegender Erfindung verwendet: Saccharomyces cerevisiae, Saccharomyces bayanus, Saccharomyces paradoxus und Saccharomyces pastorianus werden als "Saccharomyces sensu stricto" zusammengefaßt, im Gegensatz zu "Saccharomyces sensu lato", womit Saccharomyces castellii, Saccharomyces dairensis, Saccharomyces exiguus, Saccharomyces kluyveri, Saccharomyces unisporus und Saccharomyces servazzii bezeichnet werden.

Die taxonomische Zuordnung der Hefearten erfolgt vornehmlich nach morphologischen und physiologischen Kriterien (wie z.B. die Verwertung von C-Quellen, Abhängigkeit von Wuchsfaktoren, Verwertung von N-Quellen u.ä.) sowie nach dem Paarungsverhalten. Zunehmend findet auch die DNA-Analytik Verwendung bei der Erstellung von Verwandtschaftsbeziehungen zwischen Hefegattungen und Hefearten. Hingegen werden immunologische Methoden relativ selten zur Unterscheidung von Hefearten angewandt. Das liegt u.a. daran, daß Antikörper, die gegen zellwandspezifische Antigene gerichtet sind, in großem Ausmaß kreuzreagieren und dies sowohl innerhalb einer Gattung als auch über die Gattungen hinweg. Der Grund für die Kreuzreaktivität innerhalb der Gattung Saccharomyces liegt darin, daß die zellwandassoziierten Glykoproteine Kohlenhydrat-Seitengruppen tragen, die aus Mannose-Ketten aufgebaut sind, die sich von Art zu Art nicht oder kaum unterscheiden. Auch auf der Ebene der Polysaccharide (Mannane, Glucane) ist eine so hohe Homologie in der Struktur gegeben, daß eine Differenzierung mit Antikörpern nur schwer möglich erscheint.

Wie schwierig es ist, Antikörper zu erhalten, die spezifisch für eine bestimmte Hefeart sind, insbesondere S. cerevisiae, geht beispielsweise aus Kumar et al. (Infection and Immunity, 1840 (1985), pp.806-812) hervor. Die Autoren berichten, daß Antikörper, die gegen jeweils einen Vertreter der Arten Histoplasma capsulatum, Candida albicans oder Saccharomyces cerevisiae in Kaninchen hergestellt wurden, jeweils auch mit den Antigenen der beiden anderen Arten kreuzreagieren.

Vorliegender Erfindung lag demzufolge die Aufgabe zugrunde, Antikörper bereitzustellen, welche spezifisch mit einer oder wenigen Hefearten reagieren und somit die Differenzierung dieser Hefearten von anderen ermöglichen.

Die Aufgabe wurde durch die Herstellung monoklonaler Antikörper gelöst, welche überraschenderweise spezifisch die Hefearten Saccharomyces cerevisiae, Saccharomyces bayanus, Saccharomyces paradoxus und Saccharomyces pastorianus (Saccharomyces sensu stricto, s. o.) erkennen. Diese Antikörper sind gegen ein zellwandassoziiertes Antigen gerichtet und sind in verschiedenen immunologischen Nachweissystemen, wie Westernblot, Immunfluoreszenztest, Enzyme-Linked Immunosorbent Assay (ELISA), Radioimmunoassay (RIA) und Fluorescence Immunoassay (FIA) einsetzbar. Die Hybridomakultur, welche den erfindungsgemäßen Antikörper, einen murinen monoklonalen Antikörper (MAK 92-276/018) sezerniert, wurde am 22.04.1993 bei der Deutschen Sammlung von Mikroorganismen und Zellkulturen GmbH (DSM), Mascheroder Weg 1 B, 38124 Braunschweig, unter der Nummer DSM ACC2126 hinterlegt.

Vorliegende Erfindung betrifft daher den monoklonalen Antikörper MAK 92-276/018 aus dem Hybridom mit der Hinterlegungsnummer DSM ACC2126.

Die Erfindung betrifft außerdem Epitope oder immunogene Teile aus Saccharomyces sensu stricto, bevorzugt aus Saccharomyces cerevisiae, an welche der monoklonale Antikörper DSM ACC2126 bindet.

Weiterhin betrifft die Erfindung monoklonale oder polyklonale Antikörper, die an Epitope oder immunogene Teile von Saccharomyces sensu stricto, bevorzugt Saccharomyces cerevisiae, binden und welche die gleiche Antigenspezfität besitzen wie der monoklonale Antikörper DSM ACC2126. Solche Antikörper sind z.B. durch Kompetitionsversuche mit dem MAK DSM ACC2126 isolierbar.

Vorliegende Erfindung betrifft weiterhin ein Diagnostikum zur Identifizierung oder zum quantitativen Nachweis von Saccharomyces sensu stricto, enthaltend den monoklonalen Antikörper DSM ACC2126 oder einen monoklonalen oder polyklonalen Antikörper, der an Epitope oder immunogene Teile von Saccharomyces sensu stricto, bevorzugt Saccharomyces cerevisiae, bindet und welcher die gleiche Antigenspezifität besitzt, wie der monoklonale Antikörper DSM ACC2126.

Des weiteren betrifft vorliegende Erfindung ein Diagnostikum zum Nachweis von Antikörpern gegen Saccharomyces sensu stricto, enthaltend Epitope oder immunogene Teile aus Saccharomyces sensu stricto, bevorzugt Saccharomyces cerevisiae, an welche der monoklonale Antikörper DSM ACC2126 bindet.

Die Erfindung betrifft weiterhin ein Verfahren zur Herstellung monoklonaler Antikörper, dadurch gekennzeichnet, daß immunogene Teile aus Saccharomyces sensu stricto, bevorzugt Saccharomyces cerevisiae, welche mit dem monoklonalen Antikörper DSM ACC2126 reagieren, isoliert werden, daß ein geeigneter Organismus mit diesen isolierten Antigenen oder immunogenen Teilen immunisiert wird und daß aus den erhaltenen monoklonalen Antikörpern diejenigen ausgewählt werden, die für Saccharomyces sensu stricto spezifisch sind.

Außerdem betrifft vorliegende Erfindung ein Verfahren zur Identifizierung oder Quantifizierung von Saccharomyces sensu stricto in einer Probe, dadurch gekennzeichnet, daß die Probe mit einem der oben beschriebenen Saccharomyces sensu stricto-spezifischen Antikörper in Kontakt gebracht wird und danach mit geeigneten Methoden die Bildung von Antigen-Antikörper-Komplexen bestimmt wird.

Die Erfindung betrifft schließlich ein Verfahren zum Nachweis von Antikörpern gegen Saccharomyces sensu stricto in einer Probe, dadurch gekennzeichnet, daß die Probe mit den oben beschriebenen Epitopen oder immunogenen Teilen aus Saccharomyces sensu stricto in Kontakt gebracht wird und danach mit geeigneten Methoden die Bildung von Antigen-Antikörper-Komplexen bestimmt wird.

Im folgenden wird die Erfindung, insbesondere in ihren bevorzugten Ausführungsformen, detailliert beschrieben.

Die nachfolgend aufgeführten Beispiele verdeutlichen Herstellung und Verwendbarkeit der beschriebenen monoklonalen Antikörper, insbesondere des monoklonalen Antikörpers 92-276/018 (DSM ACC2126). Mit Hilfe dieses Antikörpers kann man in kurzer Zeit mit einfachen technischen Methoden Saccharomyces sensu stricto bestimmen. Identifizierungen von Hefen sind häufig notwendig in der Lebensmittel-, Bier- und Weinindustrie, aber auch zur Qualitätskontrolle in der pharmazeutischen Industrie, z.B. bei der Anzüchtung von S. cerevisiae (syn. S. boulardii) als Arzneimittel, das zur Behandlung von Diarrhöen eingesetzt wird, oder bei der Herstellung rekombinanter Proteine in S. cerevisiae.

Weitere Anwendungsmöglichkeiten für den monoklonalen Antikörper 92-276/018 finden sich im klinischen Sektor. Durch übermäßige Kolonisierung des Darmes mit Hefen kann es durch starke Alkoholbildung (gastrointestinale Alkoholfermentation) bei Patienten zu einem sogenannten ''alcohol autointoxication syndrome'' (''auto-brewery syndrome'') kommen, was zu empfindlichen gesundheitlichen Störungen der Patienten führen kann und auch von forensischem Interesse ist.

Immunsuppremierte Patienten können nach Infektion mit S. cerevisiae eine Fungämie erleiden, die im Extremfall sogar tödliche Folgen haben kann. Eine schnelle und eindeutige Identifizierung dieser Hefen mit Hilfe des monoklonalen Antikörper ermöglicht eine schnelle Therapie mit auf S. cerevisiae abgestimmten Antibiotika.

Ferner kann eine schnellere Identifizierung von Hefen mit Hilfe des monoklonalen Antikörper bei Untersuchungen durchgeführt werden, wie sie Preller et al. (J. Air Waste Managm. Ass. 39, 1094-1097 [1989]) vorgenommen haben. In einer Stadt, in deren Nähe sich eine Hefe- und eine Penicillinproduktionsanlage befanden, traten bei den Einwohnern gehäuft chronische nichtspezifische Lungenerkrankungen auf. Um zu evaluieren, ob die Hefen, in diesem Fall S. cerevisiae, ursächlich mit diesem Krankheitssymptom in Zusammenhang gebracht werden können, wurde für eine erste Bestandsaufnahme die S. cerevisiae-Partikelzahl pro Kubikmeter Luft im Bereich zwischen der Hefeproduktionsanlage und der Stadt bestimmt. Eine schnelle Identifizierung von S. cerevisiae und insbesondere ein Ausschluß von anderen Hefearten wie z.B. Rhodotorula, die häufig in der Luft zu finden ist, läßt sich mit Hilfe des monoklonalen Antikörper schnell und sicher durchführen.

### Herstellung monoklonaler Antikörper gegen das Glykoprotein gp200 aus Saccharomyces cerevisiae:

Zur Herstellung dieser Antikörper wurden sechs bis acht Wochen alte Balb/c Mäuse mit einem Glykoprotein (gp 200) immunisiert, welches von Heelan et al. (1991) Immunol. Lett. 28, pp. 181-186 beschrieben wurde. Das gp 200 kann wie folgt schnell und einfach isoliert werden: S. cerevisiae (J. Sainsbury, PLC) wird über Nacht in YPD-Medium angezogen. Die Zellen werden geerntet, zweimal in Wasser gewaschen und 10fach in Wasser resuspendiert. Die Zellsuspension wird für 1 Stunde in einem Wasserbad bei 100 °C inkubiert. Anschließend wird die Zellpräparation 5 Minuten bei 5000 UpM in der Tischzentrifuge (Varifuge 3.2 RS) zentrifugiert und der Überstand mit Centricon 100k-Filtrationssystem (Filtron) ca. 3fach angereichert. Zur Immunisierung wurden je Maus ca. 50 µg des Glykoproteins emulgiert in komplettem Freund'schen Adjuvans subcutan und in einem zweiten Fall intraperitoneal injiziert. Jeweils vier bis acht Wochen später folgte eine zweite und dritte Immunisierung ohne Adjuvants. Unmittelbar vor der eigentlichen Fusion wurden die Versuchstiere zusätzlich vier Tage hintereinander intravenös geboostert. Am Tage der Fusion wurden die Milzen steril entnommen und zu Einzelzellen suspendiert. Durch Fusion von 10⁸ Milzzellen mit 2x 10⁷ Zellen der Myelomzellinie SP 2/0 oder Zellinie X 63 Ag 8653 (J. Immunol. 173, pp. 1548-1550, [1979]) wurden Hybridzellen erzeugt, welche anschließend in einem Selektionsmedium (Dulbecco's Minimal Essential Medium, DMEM supplementiert mit 20% fötalem Kälberserum FKS, 0,1 mM Hypoxanthin, 0,4 mM Aminopterin sowie 16 mM Thymidin) auf Kulturplatten mit 24 Kavitäten (Fa. Costar) in einer Konzentration von 10⁶ Zellen/Kavität ausgesät wurden. Zwei bis drei Wochen später wurden aus den Kavitäten einzelne Zellkolonien isoliert und in jeweils eine Kavität einer neuen Kulturplatte übertragen.

Nach weiteren zwei bis drei Tagen wurden die Kulturüberstände mit Hilfe eines Enzymimmunoassays auf die Anwesenheit von gp200 spezifischen Antikörpern durchsucht. Die Überstände wurden auf gp200-beschichteten Mikrotitrationsplatten inkubiert und gegebenenfalls vorhandene spezifische Antikörper durch eine Anti-Maus/Peroxidase-Immunglobulin Konjugat-Reaktion nachgewiesen. Positive Zellinien wurden hochgezüchtet und nach Testung auf Abwesenheit von Mykoplasmen in flüssigem Stickstoff eingefroren. Parallel dazu wurden positive Zellinien mit Hilfe eines Mikromanipulators kloniert. Geeignete Klone wurden dann in Zellkultur vermehrt (Massenkultur in Rollerflaschen). Die Reinigung erfolgte über Ammoniumsulfatfällung sowie Protein A-Chromatographie. Die Reinheit wurde mittels HPLC sowie Gelelektrophorese überprüft, die Immunglobulinklassen durch Immundiffusion nach Ouchterlony bestimmt.

### Beispiel 1

Hybridom-Überstände werden auf ihre Reaktivität in Western-Blots mit Antigen aus Saccharomyces cerevisiae und Schizosaccharomyces pombe als Vergleich getestet.

Dazu wurden S. cerevisiae (VLSF 07158) und S. pombe (CBS 1043) 3 Tage in YPD-Medium (1% Hefeextrakt, 2% Pepton, 2% Glucose) in 250 ml Erlenmeyerkolben auf einem Schüttelgerät bei 180 UpM und bei 30 °C gezüchtet. Nach Zentrifugation von jeweils 10 ml Kulturbrühe wurden die Zellen geerntet, in 1 ml 10 mM Tris-Puffer (pH 7,5) aufgenommen, mit Glaskugeln versetzt und in einer Glaskugelmühle aufgeschlossen. Anschließend wurde das Homogenat nach Laemmli (Nature 227, 680-685 [1970]) erhitzt, in einem 10%igen SDS-Polyacrylamidgel aufgetrennt und auf Nitrozellulosemembranen transferiert (Burnette, Anal. Biochem. 112, 195-203 [1981]). Die Nitrozellulosestreifen wurden dann in Tris-Puffer (10 mM Tris-HCl, pH 7,5, 150 mM NaCl, 1% Gelatine, 4% Tween 20), die jeweils monoklonale Antikörper in einer Konzentration von ca. 10 µg/ml von neun verschiedenen Hybridomaüberständen enthielten, inkubiert. Als Konjugat wurden anti-Maus Antikörper verwendet, die mit alkalischer Phosphatase gekoppelt waren. Als Substrate dienten Fast Blau 19 Salz (Fa. Serva, Heidelberg) und Naphtol AS-MX-Phosphat (Fa. Sigma Chemie, München). Zur Bestimmung der relativen Molmasse wurden auf dem SDS-Polyacrylamidgel zusätzlich Rainbow™ Protein Molekulargewichtsmarker (Amersham Buchler GmbH, Braunschweig) aufgetragen.

Alle neun Antikörperspezifitäten erkannten im Western blot Antigene aus S. cerevisiae.

Von neun getesteten Überständen waren vier nur sehr schwach positiv:
- 92 - 285/18
- 92 - 285/27
- 92 - 285/29
- 92 - 275/02
Vier Überstände zeigten eine recht gute Reaktivität:
- 92 - 285/5
- 92 - 285/10
- 92 - 285/15
- 92 - 285/16
Die stärkste Reaktivität zeigte der Überstand
- 92 - 276/018.

Keiner der neun Überstände erkannte hingegen ein Antigen aus S. pombe. Dies wurde als Kriterium für eine spezifische Immunoreaktivität der monoklonalen Antikörper gewertet.

Im weiteren wurde die Reaktivität des monoklonalen Antikörpers 92 - 276/018 eingehender untersucht.

### Beispiel 2

Die Spezifität des monoklonalen Antikörpers 92 - 276/018 (DSM ACC2126) wurde einerseits in Western blots und andererseits an ganzen Hefezellen in der Immunfluoreszenz analysiert.

Die Immunfluoreszenz an ganzen Hefezellen mit monoklonalen Antikörpern wurde wie folgt durchgeführt. Die Hefen wurden in Medien aufgezogen, wie es für die Westernblot Analysen beschrieben ist. In die Vertiefungen von Immunfluoreszenzglasplättchen wurden jeweils 10 µl einer Hefezellensuspension eingetragen. Nach 1 Stunde Inkubation bei RT wurde der Überstand abgesaugt und die Zellen durch Zugabe von 4°C kaltem Aceton fixiert. Anschließend wurde mit Wasser gespült und getrocknet. Es wurden jeweils 10 µl der Antikörperlösung (100 µg/ml) in die Vertiefungen pipettiert. Nach einer Stunde Inkubation bei 37°C in einer feuchten Kammer wurde der Überstand abgezogen, dreimal gewaschen und FITC-markiertes anti-Maus-Immunglobulinkonjugat zugesetzt.

Nach einstündiger Inkubation und anschließendem Waschen wurde die mikroskopische Analyse vorgenommen.

Der MAK 92 - 276/018 wurde hinsichtlich seiner Reaktivität mit verschiedenen Hefearten untersucht. Die verschiedenen Hefearten wurden von folgenden Institutionen bezogen: Deutsche Sammlung von Mikroorganismen und Zellkulturen GmbH (DSM), Braunschweig, BRD; Centraalbureau voor Schimmelcultures (CBS), Baarn-Delft, NL; Versuchs- und Lehranstalt für Spiritusfabrikation und Fermentationstechnologie in Berlin (VLSF), Berlin, BRD; Institut für Mikrobiologie und Weinforschung (IMW) der Johannes-Gutenberg-Universität Mainz, BRD, Yeast Genetic Stock Center (YGSC), Berkeley, USA und J. Sainsbury PLC, London, England. Darüber hinaus wurden einige Hefestämme von Forschungsinstituten bezogen, die nur Stammbezeichnungen aufweisen, die in der Literatur zitiert werden, aber keiner offiziellen Institutsnomenklatur unterworfen wurden. Folgende Hefearten gelangten zum Einsatz:
- Yarrowia lipolytica: DSM 1345
- Debaromyces hansenii: DSM 3428
- Hanseniaspora guilliermondii: DSM 3432
- Schwanniomyces occidentalis: DSM 3451
- Lipomyces starkeyi: DSM 70295
- Pichia pastoris: DSM 70382
- Zygosaccharomyces bailii: DSM 70492
- Saccharomycodes ludwigii: DSM 3447
- Torulaspora delbrueckii: DSM 70504
- Torulaspora pretoriensis: DSM 70525
- Hanseniaspora uvarum: IMW 473
- Schizosaccharomyces pombe: CBS 1043
- Dekkera anomala: CBS 4210
- Kluyveromyces marxianus: CBS 369
- Pachysolen tannophilus: CBS 4045
- Candida boidinii: CBS 5777
- Candida albicans: CBS 2730
In diesem Kollektiv von Hefearten sind auch vier Arten vertreten, die früher der Gattung Saccharomyces zugeordnet waren:
- Kluyveromyces marxianus: ex Saccharomyces fragilis
ex Saccharomyces lactis
- Zygosaccharomyces bailii: ex Saccharomyces elegans
- Torulaspora delbrueckii: ex Saccharomyces fermentati
- Torulaspora pretoriensis: ex Saccharomyces pretoriensis
Diese vier Hefearten, die aufgrund morphologischer und physiologischer Kriterien seinerzeit der Gattung Saccharomyces zugeordnet waren und hinsichtlich der taxonomischen Kriterien mit der Gattung Saccharomyces eine hohe Ähnlichkeit aufweisen, lassen sich also immundiagnostisch klar von Saccharomyces cerevisiae mit Hilfe des monoklonalen Antikörpers 92-276/018 abgrenzen.

Keine von diesen Hefearten aus verschiedenen Familien reagierte mit dem monoklonalen Antikörper 92 - 276/018, weder in der Immunfluoreszenz noch in Western blot-Analysen.

### Beispiel 3

Im weiteren wurde die Reaktivität des monoklonalen Antikörpers 92 - 276/018 mit verschiedenen Wild-Isolaten der Art S. cerevisiae untersucht.
Folgende Stämme von S. cerevisiae kamen zum Einsatz:
S. cerevisiae DSM 70471 (ex S. ellipsoideus)
S. cerevisiae DSM 70478 (ex S. chevallieri)
S. cerevisiae DSM 70487 (ex S. diastaticus)
S. cerevisiae DSM 70514 (ex S. italicus)
S. cerevisiae CBS 5926 (ex S. boulardii)
S. cerevisiae IMW 116
S. cerevisiae IMW 188
S. cerevisiae IMW 25
S. cerevisiae IMW 92
S. cerevisiae VLSF 07158
S. cerevisiae J. Sainsbury, PLC
S. cerevisiae YGSC X2180 1A
All diese Stämme reagierten mit dem monoklonalen Antikörper sowohl im Western blot als auch in der Immunfluoreszenz. Die ersten vier untersuchten Stämme waren vormals anderen Saccharomyces-Arten zugeordnet worden, die nun keine allgemeine Akzeptanz mehr finden und alle der Art S. cerevisiae zugeführt worden sind.

### Beispiel 4

Im weiteren wurde die Reaktivität des monoklonalen Antikörpers mit verschiedenen Laborstämmen der Art S. cerevisiae untersucht. In der folgenden Tabelle sind die untersuchten Stämme, ihre genetischen Marker und ihre Reaktivität mit dem monoklonalen Antikörper aufgeführt.

**Tabelle**

| **Reaktivität des monoklonalen Antikörpers 92-276/018 mit Saccharomyces cerevisiae Laborstämmen** | | | |
|---|---|---|---|
| **Stamm** | **genetische Marker** | **Immunblot** | **Immunofluoreszenz** |
| Cl3ABYS86 | ura3-2,leu2,his,pra1,prb1,prc1,cps1 | + | + |
| HT 393 | ura3,leu2,pra1,prb1,prc1,cps1,pre1 | + | + |
| 79 | leu2,trp1 | + | + |
| S150-2B | leu2-3,leu2-112,ura3-52,trp1-289,his3Δ1 | + | + |
| TY 4 | leu2,ura3,ts1 | + | + |
| 4STLU | leu2,ura3,ts1,srb1 | + | + |
| CGY 1465 | leu2,ura3,ssc1/pmr1 | + | + |
| A 258 | can1,leu2,his4,met14,trp1,ura3,ose1,SUC2 | + | + |
| A 259 | can1,leu2,his4,met14,trp1,ura3,OSE1,SUC2 | + | + |

Unabhängig von den genetischen Markern reagierten alle Stämme. Auch S. cerevisiae-Stämme mit einer veränderten Zellwandstruktur oder Deletionen von Proteinen im periplasmatischen Raum aufgrund von genetischen Mutationen wie z.B. 4STLU, CGY 1465, TY 4 und A 258 wurden von dem monoklonalen Antikörper erkannt.

Die hier aufgeführten S. cerevisiae-Laborstämme gelangen vorwiegend zu gentechnischen Zwecken zur Anwendung, insbesondere dienen sie als Wirtszellen zur Expression von heterologen Proteinen. Die Stämme sind frei zugänglich und in der Literatur hinsichtlich ihrer Marker und möglichen Verwendbarkeit beschrieben. Im einzelnen sind dies:
- A 258 und A 259:: Sakai et al., Genetics 119, pp. 499-506 [1988].
- CGY 1465:: Rudolph et al., Cell 58, pp. 133-145 [1989].
- Cl3ABYS86 und HT 393:: Heinemeyer et al., EMBO J. 10, pp. 535-562 [1991].
- 4STLU und TY 4:: Waltschewa et al., Yeast 5, pp. 313-320 [1989].
- S150-2B:: Baldari et al., EMBO J. 6, pp. 229-234 [1987].
- 79:: Price et al., Gene 55, pp. 287-293 [1987].

### Beispiel 5

Nach Barnett werden heute der Gattung Saccharomyces zehn Arten zugeordnet. Es wurde nun untersucht, ob der monoklonale Antikörper 92-276/018 außer mit S. cerevisiae auch mit den anderen neun Arten reagiert.

Der monoklonale Antikörper 92-276/018 zeigte keine Reaktion im Westernblot und in der Immunfluoreszenz mit folgenden Saccharomyces-Arten:
- S. exigus: CBS 134
- S. dairensis: CBS 421
- S. unisporus: CBS 398
- S. servazzii: CBS 4311
- S. castelli: CBS 4309
- S. kluyveri: CBS 2861
Der monoklonale Antikörper erkannte aber die Arten:
- S. bayanus: DSM 70412
- S. bayanus: DSM 70511
- S. bayanus: DSM 70547
- S. pastorianus: DSM 6580
- S. pastorianus: CBS 1260
- S. paradoxus: CBS 406
- S. paradoxus: CBS 2980
- S. paradoxus: CBS 432
Es zeigt sich also, daß Antikörper gegen das gp 200 von S. cerevisiae und insbesondere der monoklonale Antikörper 92-276/018 geeignete Mittel darstellen, die Art S. cerevisiae zu identifizieren bzw. eine andere Hefeart als S. cerevisiae dahingehend zu charakterisieren, daß man diese als S. cerevisiae ausschließen kann.

Wenn nun der monoklonale Antikörper 92-276/018 nicht nur S. cerevisiae erkennt, sondern auch die Arten S. paradoxus, S. pastorianus und S. bayanus, so ist dies keineswegs als Unspezifität anzusehen. Umfangreiche DNA-Studien haben ergeben, daß S. paradoxus und S. bayanus einen äußerst hohen Verwandtschaftsgrad mit S. cerevisiae aufweisen (Naumov et al., Yeast 8, 599-612 [1992]). Darüber hinaus wird die Art S. pastorianus als ein Hybrid aus den Arten S. cerevisiae und S. bayanus angesehen. Die hohe Ähnlichkeit zwischen den hier genannten Arten führte Naumov et al. dazu, diese Saccharomyces-Arten als Geschwisterarten von S. cerevisiae zu bezeichnen.

Im Zusammenhang mit der Tatsache, daß die Kriterien und deren Gewichtung, die bei der Bestimmung von Hefearten zu Grunde gelegt werden, zeitlichen Schwankungen und Änderungen unterworfen sind, könnte man auch argumentieren, daß die Arten S. paradoxus, S. pastorianus und S. bayanus gar nicht als eigenständige Arten zu sehen sind, sondern vielmehr als Varietäten von S. cerevisiae. Aber auch dann, wenn man die von Barnett aufgestellte Klassifikation der Arten innerhalb der Gattung Saccharomyces gelten läßt, hat der monoklonale Antikörper einen großen Wert; denn er identifiziert ein Hefeisolat bis zur Gattung Saccharomyces und schließt mehrere Arten innerhalb dieser Gattung aus.

### Beispiel 6

Wenn man Nitrozellulosepapier, auf das Proteine von S. cerevisiae-Zellextrakten von Polyacrylamidgelen transferiert worden sind, in Perjodatlösungen inkubiert und anschließend die verbliebene Reaktivität mit dem monoklonalen Antikörper 92-276/018 untersucht, so stellt man fest, daß nach einer 30minütigen Inkubation in Gegenwart von 0,1 mM Perjodat noch die volle Reaktivität vorhanden ist. Bei Inkubation der Nitrozellulosestreifen in 10 mM oder 50 mM Perjodat konnte hingegen keine Bindung des monoklonalen Antikörpers 92-276/018 nachgewiesen werden.

Führt man die Inkubation des monoklonalen Antikörpers mit der mit S. cerevisiae-Antigenen beschichteten Nitrozellulose in Gegenwart von 100 mM Methyl-α-D-Methyl-Mannosid aus, so ist die Reaktivität stark beeinflußt. In Gegenwart von Methyl-α-D-Methyl-Glucanosid ist dagegen die Reaktivität gegenüber der Kontrolle, in der keine Zucker zugesetzt sind, unvermindert.

Aus diesen zwei Befunden kann geschlossen werden, daß die antigenen Determinanten, die von dem monoklonalen Antikörper 92-276/018 erkannt werden, wahrscheinlich Kohlenhydratstrukturen darstellen, oder Peptidstrukturen, deren Affinität zu dem Antikörper durch Kohlenhydratseitenketten beeinflußt wird.

In Westernblot-Analysen wird durch den monoklonalen Antikörper 92-276/018 nicht nur Antigen erkannt, das mit einem scheinbaren Molekulargewicht von 200 kDal in SDS-Gel bandet, sondern die spezifische Reaktivität zieht sich über einen größeren Bereich von ca. 40 kDal bis 200 kDal hin. Dies deutet darauf hin, daß Mannoproteine oder Mannane mit einer großen Heterogenität im Molekulargewicht erkannt werden. Es ist wahrscheinlich, daß der monoklonale Antikörper ein Kohlenhydrat-spezifisches Epitop erkennt, das Bestandteil mehrerer Glycoproteine oder Mannanstrukturen sein kann und das gp200 nur ein Antigen von mehreren von S. cerevisiae mit solch einem Epitop darstellt.

## Patentansprüche

1. Hybridom DSM ACC2126, welches den monoklonalen Antikörper DSM ACC2126 sezerniert.

2. Monoklonaler Antikörper aus DSM ACC2126

3. Epitope oder immunogene Teile aus Saccharomyces sensu stricto, an welche der monoklonale Antikörper DSM ACC2126 bindet.

4. Monoklonale oder polyklonale Antikörper, die an Epitope oder immunogene Teile gemäß Anspruch 3 binden und die die gleiche Antigenspezifität besitzen, wie der monokonale Antikörper aus DSM ACC 2126.

5. Epitope oder immunogene Teile nach Anspruch 3, dadurch gekennzeichnet, daß sie aus Saccharomyces cerevisiae stammen.

6. Diagnostikum zur Identifizierung und Quantifizierung von Saccharomyces sensu stricto, enthaltend Antikörper nach Anspruch 2 oder 4.

7. Diagnostikum zum Nachweis von Antikörpern gegen Saccharomyces sensu stricto, enthaltend Epitope oder immunogene Teile nach Anspruch 3.

8. Verfahren zur Herstellung von monoklonalen Antikörpern gemäß Anspruch 4, dadurch gekennzeichnet, daß
(a) Epitope oder immunogene Teile aus Saccharomyces sensu stricto, welche mit dem monoklonalen Antikörper DSM ACC2126 reagieren, isoliert werden;
(b) ein geeigneter Organsimus mit den isolierten immunogenen Teilen immunisiert wird;
(c) aus den erhaltenen monokonalen Antikörpern diejenigen ausgewählt werden, die für Saccharomyces sensu stricto spezifisch sind.

9. Verfahren zur Identifizierung oder Quantifizierung von Saccharomyces sensu stricto in einer Probe, dadurch gekennzeichnet, daß
a) die Probe mit einem Antikörper nach Anspruch 2 oder 4 in Kontakt gebracht wird;
b) mit geeigneten Methoden die Bildung von Antigen-Antikörperkomplexen bestimmt wird.

10. Verfahren zum Nachweis von Antikörpern gegen Saccharomyces sensu stricto in einer Probe, dadurch gekennzeichnet, daß
a) die Probe mit Epitopen oder immunogenen Teilen nach Anspruch 3 in Kontakt gebracht wird;
b) mit geeigneten Methoden die Bildung von Antigen-Antikörperkomplexen bestimmt wird.
